# DEMANDE DE BREVET EUROPEEN

(11) **EP 1 371 354 A1**
(43) Date de publication de la demande: **17.12.2003**
(21) Numéro de dépôt: 03291162.0
(22) Date de dépôt: 19.05.2003
(51) Int. Cl.: A61K 7/06

(54) **Composition cosmétique destinée à conferer du volume aux fibres kératiniques et utilisation cosmétique de nanotubes pour conférer du volume aux fibres kératiniques**

(30) Priorité: 06.06.2002 FR 0206967
(71) Demandeur: L'OREAL, 75008 Paris (FR)
(72) Inventeur: Giroud, Franck, 92110 Clichy (FR); Favreau, Valérie, 95150 Taverny (FR)
(74) Mandataire: Bourdeau, Françoise

(57) **Abrégé**

L'invention concerne une composition cosmétique destinée à conférer du volume aux fibres kératiniques, caractérisée en ce qu'elle comprend en association avec un milieu cosmétiquement acceptable des nanotubes paticuliers, ainsi que l'utilisation cosmétique de ces nanotubes pour conférer du volume aux fibres kératiniques.

## Description

La présente invention est relative à de nouvelles compositions destinées à conférer du volume aux fibres kératiniques, et notamment aux cheveux, comprenant des nanotubes, ainsi qu'à l'utilisation cosmétiqué de nanotubes pour conférer du volume aux fibres kératiniques.

Il existe de nombreux produits de coiffage permettant d'apporter du volume aux cheveux. Un inconvénient lié à ces produits, le plus souvent à base de polymères filmogènes, réside dans le fait que l'effet cosmétique disparaît dès le premier shampooing.

On connaît par ailleurs des traitements permanents des fibres kératiniques. Ces traitements utilisent un agent réducteur et un agent oxydant, et nécessitent une mise sous tension mécanique des cheveux à l'aide d'un matériel d'enroulage, afin de conférer une mise en forme durable de la chevelure.

Ces procédés, qui permettent effectivement d'augmenter le volume de la chevelure, présentent toutefois l'inconvénient de modifier le niveau de frisure de la chevelure et de dégrader le toucher de la fibre.

Il apparaît ainsi nécessaire de développer des compositions permettant d'accroître le volume de la chevelure sans modifier la forme ou le toucher des cheveux.

La présente invention propose de nouvelles compositions, permettant de remédier à ces inconvénients.

En particulier, la Demanderesse vient de découvrir de façon surprenante qu'une composition comprenant des nanotubes permet d'apporter du volume à la coiffure, sans altération du toucher des cheveux, ni de leur forme, sans dégradation de la fibre et sans adhésion des cheveux entre eux par une matière filmogène. En outre, ces propriétés cosmétiqués sont rémanentes aux shampooings.

La présente invention a donc pour objet une composition cosmétique destinée à conférer du volume aux fibres kératiniques, comprenant des nanotubes.

L'invention a également pour objet l'utilisation cosmétique de nanotubes pour conférer du volume à des fibres kératiniques.

D'autres objets de l'invention apparaîtront à la lecture de la description et des exemples qui suivent.

Les compositions cosmétiques conformes à l'invention sont essentiellement caractérisées par 1e fait qu'elles comprennent en association avec un milieu cosmétiquement acceptable des nanotubes constitués d'au moins un élément appartenant aux groupes II_{A}, III_{A}, IV_{A}, V_{A}, VIII, I_{B}, II_{B}, III_{B}, VI_{B} et VII_{B} de la classification périodique des éléments.

On entend par nanotubes des nano-objets dont l'organisation des atomes ou des molécules confère à la nano-structure une forme de tube. Ces nanotubes peuvent être à simple paroi ou multi-parois.

Le diamètre des nanotubes est classiquement compris entre 1 et 300 nm et la longueur des nanotubes est classiquement comprise entre 10 nm et 10 mm.

A titre d'exemples d'éléments constitutifs des nanotubes de l'invention, on peut citer le carbone, le silicium, le tungstène, l'argent, l'or, le bore, le zinc, le platine, le magnésium, le fer, le cérium et l'aluminium.

De préférence, les nanotubes sont constitués d'au moins un élément appartenant au groupe IV_{A} de la classification périodique des éléments. Encore plus préférentiellement, ledit élément est le carbone.

Lorsqu'un des éléments constitutifs des nanotubes est le carbone, lesdits nanotubes peuvent être constitués totalement ou partiellement de molécules organiques. A titre d'exemple de molécules organiques on peut citer les phospholipides diacétyléniques, les glutamates, les diamides à longue chaîne, les glucophospholipides, les alkylphénylglucopyranosides.

Préférentiellement, le squelette des nanotubes est constitué uniquement d'atomes de carbone.

Les figures 1 à 6 annexées représentent différents nanotubes de carbone utilisés selon l'invention.

Ces nario-formes carbonées sont classiquement obtenues par sublimation de graphite à très haute température par l'intermédiaire d'un arc électrique. Les nanotubes de carbone peuvent être formés par un seul plan de graphène ; dans ce cas ils sont dits à simple paroi (Single Wall NanoTube SWNT en anglais). La figure 1 représente un nanotube à simple paroi. L'enroulement des plans de graphène peut être en zig-zag, créneau ou chiral. Les nanotubes peuvent être également constitués par plusieurs tubes " emboîtés " les uns dans les autres ; dans ce cas ils sont dits nanotubes multi-parois (Multi Wall NanoTube MWNT en anglais).

Selon un mode de réalisation de l'invention, afin d'obtenir une solubilisation ou une exfoliation optimale des nanotubes de carbone dans le milieu cosmétique considéré, 1a surface des nanotubes est fonctionnalisée.

Par l'expression " fonctionnalisée ", on entend selon l'invention la présence de groupements fonctionnels pouvant interagir physiquement ou chimiquement entre eux ou avec le milieu extérieur.

Tous mécanismes réactionnels peuvent être utilisés pour fonctionnaliser les plans de graphène constituant les nanotubes de carbone. A titre d'exemple, la fonctionnalisation des nanotubes de carbone peut être réalisée par un mécanisme réactionnel du type substitution nucléophile, substitution électrophile, substitution radicalaire, addition, élimination, réarrangement, oxydation, réduction, réaction acido-basique, réaction électrochimique, ou encore réaction photochimique.

Parmi les fonctions pouvant être greffées à la surface des plans de graphène constituant le nanotube de carbone, on peut citer les groupements carboxyliques. La figure 2 représente un nanotube à simple paroi à la surface duquel sont greffés des groupements carboxyliques. Cette fonctionnalisation est décrite dans l'article *" Solution Properties of Single Walled Carbone Nanotube ", J. Chen et Coll (Science 1998, vol 282, n° 5396, pages 95-98).*

On peut également citer la solubilisation des nanotubes dans un isolvant polaire tel que eau, éthanol, par oxydation des plans de graphène par un mélange HCl/CrO₃, décrite dans l'article *" Room Température Filling of Single Wall Carbon NanotubesWith Oxide in open air ", J. Mittal et Coll. (Chem. Phys. Lett. 2001, vol 339, n°5-6, pages 311-318)* ou encore par condensation sur les nanotubes d'un acide aminé et d'un aldéhyde *(J. Am. Soc., vol 124, n°5, 2002, pages 760 et 761).*

Des fonctions hydrophobes peuvent également être greffées à la surface des plans de graphène constituant le nanotube de carbone. On peut par exemple citer la fluoration des nanotubes de carbone décrite dans l'article *" Fluorinated Single wall nanotubes ", K.N. Kudin et Coll. (Phys. R-ev. B63, 45413).*

On peut également greffer des molécules inorganiques telles que les alcoxysilanes *(Nano. Lett., vol 2, n°4, 2002 pages 329 à 332).*

La fonctionnalisation des plans de graphène peut s'effectuer en plusieurs étapes, comme par exemple la fonctionnalisation des nanotubes de carbone par des amides à chaîne grasse, décrite dans l'article *" Dissolution of Single Wall Carbon Nanotube ", M. A. Hamon et* coll. *(Adv. Mater. 1999, 11, n°10).* La figure 3 représente un nanotube de carbone à simple paroi dont la surface est fonctionnalisée par la fonction amide à chaîne grasse de formule CONH-4-C₆H ₄(CH₂)₁₃CH₃. Cette fonctionnalisation en plusieurs étapes peut également conduire au greffage du glucose *(Nano. Lett., vol 2, n°4, 2002 pages 369 à 373).*

La fonctionnalisation des nanotubes de carbone peut être réalisée par de simples molécules, mais également par des oligomères, des polymères ou des dendrimères. On peut citer à titre d'exemple l'article *" A New Purification Method for Single Wall Carbon Nanotubes ", M. Hoizinger et Coll (Appl. Phys. A 70 (2000) 599),* qui décrit le greffage de structures dendritiques à la surface des plans de graphène constituant le nanotube de carbone. La figure 4 représente un nanotube de carbone à simple paroi dont la surface est fonctionnalisée par des structures dendritiques.

Outre l'amélioration de la dispersion des nanotubes de carbone dans les milieux cosmétiques, la fonctionnalisation de la surface peut également être réalisée afin d'augmenter l'affinité des nanostructures carbonées pour la matière kératinique. L'amélioration de l'affinité entre les nanotubes et la matière kératinique induite par la fonctionnalisation des plans de graphène peut être 1e fruit de l'accroissement des interactions de type Van der Waals et/ou le fruit de l'apparition de liaions hydrogène et/ou ionique. Ainsi, le ou les groupements fonctionnels sont susceptibles de créer avec les fibres kératiniques une ou plusieurs liaisons chimiques choisies parmi les interactions de type Van der Waals, les liaisons hydrogène, les liaisons ioniques et les liaisons covalentes. Dans ce cadre on peut citer le greffage de molécules cationiques à la surface de nanotubes de carbone, décrit dans l'article *" Exohedral Sidewall Reactions of Single Walled Carbon Nanotubes in Molecular Nanostructures ", M. Holzinger et ail (Proceeding of the XIIth International Winterschool on Electronic Properties of Novel Materials :Molecular Nanostructures, Kirchberg, Austria, March 2001).* Dans le cadre de greffage de molécules cationiques on peut aussi mentionner le greffage de dérivés de polyéthylèneimine *(Nano. Lett., vol 2, n°3, 2002 pages 231 à 234).* La figure 5 représente un nanotube de carbone à simple paroi dont la surface est fonctionnalisée par des molécules cationiques.

Pour accroître l'affinité des nanostructures carbonées pour la matière kératinique, il est également possible de greffer de façon covalente la nanostructure carbonée sur la fibre kératinique. Pour ce faire, la fonctionnalisation des plans de graphène est réalisée à l'aide de groupements possédant une certaine réactivité sur les acides aminés constituant la matière kératinique. De préférence, le ou les groupements fonctionnels susceptibles de créer avec les fibres kératiniques une ou plusieurs liaisons chimiques covalentes sont choisis parmi les groupements susceptibles de réagir avec les thiols, les disulfures, les acides carboxyliques, les alcools et les aminés. De prférence, le ou les groupements fonctionnels susceptibles de réagir avec les thiols, les disulfures, les acides carboxyliques, les alcools et les aminés sont choisis parmi :
- les époxydes,
- les groupements à cycle aziridine,
- les groupements vinyle et vinyle activé, tels que acrylonitrile, esters acryliques et méthacrylique, acide et ester crotonique, acides et esters cinnamique, styrène et dérivés, butadiène, éthers de vinyle, vinyle cétone, esters maléiques, maléimides, vinyl sulfones, ou vinyle cyanoacrylate,
- les acides carboxyliques et leurs dérivés comme les fonctions anhydride, chlorure d'acide, esters,
- les acétals, hémiacétals,
- les aminals, hémi-aminals,
- les cétones et alpha-hydroxycétones, alpha-halocétones,
- les lactones, thiolactones,
- l'isocyanate,
- le thiocyanate,
- imines,
- les imides (succinimides, glutimides), tels que le N-hydroxysuccinimide ester,
- les imidates,
- l'oxazine et 1'oxazoline,
- l'oxazinium et l'oxazolinium,
- les halogénures d'alkyle ou d'aryle ou d'aralkyle, l'halogène étant choisi parmi l'iode, le brome ou le chlore,
- les halogénures de cycle insaturé, le cycle étant un cycle carboné ou un hétérocycle, tels que le chlorotriazine, le chloropyrimidine, le chloroquinoxaline, le chlorobenzotriazole,
- les halogénures de sulfonyle de formule RSO₂X, R étant un groupement alkyle, et X étant choisi parmi le fluor et le chlor,
- les dérivés du silicium tels que les alcoxysilanes, les silanols.

Afin d'obtenir une dispersion (exfoliation) optimale des nanotubes de carbone dans le mileu cosmétique considéré, la composition contient de préférence au moins un tensio-actif. Le tensio-actif peut être choisi parmi les molécules amphiphiles, les oligomères amphiphiles, les plusieurs dendrimères amphiphiles, les polymères amphiphiles et leurs mélanges.

Parmi les divers groupements hydrophobes des tensioactifs sont préférés les groupements capables de s'adsorber par " π-stacking " (recouvrement des nuages électroniques des noyaux aromatiques) à 1a surface des plans de graphène constituant le nanotube de carbone. Parmi les molécules aptes à générer une telle interaction sont préférées les molécules aromatiques telles que le styrène et ses dérivés ou le pyrène et ses dérivés.

Quant aux groupements .et/ou aux blocs composant la partie hydrophile du tensio-actif, ils peuvent être de nature non ionique, amphotère, zwitterionique, anionique ou cationique.

Les groupements hydrophiles sont de préférence choisis afin d'améliorer l'affinité de la surface des nanotubes pour la matière kératinique, que ce soit par l'accroissement des interactions de type Van der Waals et/ou la création de liaisons hydrogène et/ou ionique. Les groupements hydrophiles sont de préférence de nature cationique.

Afin d'accroître l'affinité des nanotubes pour la matière kératinique, le ou les tensio-actifs peuvent également être susceptibles de créer avec les fibres kératiniques une ou plusieurs liaisons chimiques covalentes, par exemple par réaction sur les fonctions disulfures, thiols, acides carboxyliques, aminés et alcools des acides aminés composant la matière kératinique. On peut citer à titre d'exemple l'utilisation de systèmes tensio-actif réactifs du type pyrène succinimidyl ester décrits dans l'article " *Noncovalent Sidewall Functionalization of Single Walled Carbon Nanotubes for Protein Immobilisation ", J. Chen et Coll. (J. Am. Chem. Soc., 123, (16), 3838-3839, 2001).* Les nanotubes de carbone peuvent ainsi être stabilisés par l'acide 1-pyrène butanoïque succinimidyl ester. La fonction succinimidyl ester est capable de réagir à température ambiante sur les fonctions aminés de la matière kératinique. La figure 6 représente un nanotube de carbone à simple paroi dont la surface est fonctionnalisée par l'acide 1-pyrène butanoïque succinimidyl ester.

Certains saccharides, et plus particulièrement les saccharides cycliques comme les cyclodextrines possèdent également un excellent pouvoir dispersant vis-à-vis des nanostructures carbonées *(J. Am. Chem. Soc., 123, 6201, (2001)).* Les polysaccharides hyperbranchés, tels les arabinogalactans comme la gomme arabique favorisent également l'exfoliation dans l'eau des nanotubes de carbone *(Nano Lett. 1, 12, 697-702, 2001).*

- Les nanotubes peuvent être présents dans la composition cosmétique dans des proportions en poids comprises entre 0,00001% et 30%, de préférence entre 0,0001% et 5%, et encore de préférence entre 0,001% et 1% du poids total de la composition.

Le milieu cosmétiquement acceptable de la solution comprenant les nanotubes est constitué de préférence par :
- de l'eau,
- des alcools aliphatiques ou aromatiques, de préférence l'éthanol, l'alcool benzylique, les alcools gras, les polyols modifiés ou non tels que le glycérol, le glycol, le propylène glycol, le dipropylène glycol, le butylène glycol, le butyle diglycol,
- des silicones, volatiles ou non,
- des huiles minérales, organiques ou végétales,
- des cires oxyéthylénées ou non, des paraffines, des alcanes et de préférence des alcanes de C₅ à C₁₀,
- des acides gras, des amides grasses, des esters gras et plus particulièrement des benzoates ou des salicylates d'alcool gras,
- de l'acétone, de la méthyléthylcétone, de l'acétate de méthyle, de l'acétate de butyle, de l'acétate d'étyle, du diméthoxyéthane, du diéthoxyéthane,
- ainsi que leurs mélanges.

Le milieu cosmétiquement acceptable peut se présenter tel quel ou sous forme émulsionnée ou encapsulée.

Les compositions conformes à l'invention peuvent en outre contenir un propulseur. Le propulseur est constitué par les gaz comprimés ou liquéfiés habituellement employés pour la préparation de compositions aérosols. On emploie de préférence l'air, le gaz carbonique, l'azote comprimé ou encore un gaz soluble comme le diméthyléther, les hydrocarbures halogénés (en particulier fluorés) ou non, ainsi que leurs mélanges.

La composition peut également contenir des additifs cosmétiques usuels choisis dans la liste non exhaustive comprenant les agents réducteurs, les agents oxydants, les corps gras, les silicones, les agents épaississants, les adoucissants, les agents anti-mousse, les agents hydratants, les agents émollients, les agents alcalinisants, les plastifiants, les filtres solaires, les colorants directs ou d'oxydation, les pigments, les charges minérales, les argiles, les minéraux colloïdaux, les nacres, les parfums, les peptisants, les conservateurs, les polymères fixants ou non, les polymères conditionneurs, les protéines, les vitamines.

Après application sur la chevelure, la composition peut être rincée ou non. Elle peut se présenter sous diverses formes galéniques telles qu'une lotion, un spray, une mousse, un shampooing ou un après-shampooing.

L'invention a également pour objet l'utilisation cosmétique de nanotubes pour conférer du volume à des fibres kératiniques. Les nanotubes peuvent être tels que définis ci-dessus.

L'exemple suivant est destiné à illustrer l'invention sans pour aùtant présenter un caractère limitatif.

### Exemple : démonstration de l'effet de volume durable conféré aux fibres kératiniques

1g de composition de l'invention et 1g de composition témoin sont appliqués sur les cheveux (2,7 g de cheveux européens châtains de 20 cm de long environ). Après 2 minutes de pose, la chevelure est rincée à l'eau, puis mise en forme au sèche-cheveux.

| Composition témoin | |
|---|---|
| Cyclopentasiloxane (1) | 10% m.a. |
| Huile de ricin hydrogénée oxyéthylénée (7OE) (2) | 10% m.a. |
| Diméthicone copolyol (3) | 0,5% m.a. |
| Propylène glycol | 2,5% m.a. |
| Behentrimonium chloride (4) | 1,2% m.a. |
| Eau | Qsp 100% |

| Composition de l'invention | |
|---|---|
| Cyclopentasiloxane (1) | 10% m.a. |
| Huile de ricin hydrogénée oxyéthylénée (7OE)(2) | 10% m.a. |
| Diméthicone copolyol (3) | 0,5% m.a. |
| Propylène glycol | 2,5% m.a. |
| Behentrimonium chloride (4) | 1,2% m.a. |
| Nanotubes de carbone simple paroi(5) | 0,01% m.a. |
| Eau | Qsp 100% |

| | |
|---|---|
| (1) DC 245 FLUID commercialisé par la société DOW CORNING | |
| (2) ARLACEL 989 commercialisé par la société UNIQUEMA | |
| (3) DC 2-5225 C commercialisé par la société DOW CORNING | |
| (4) GENAMIN KDM-F comercilisé par la société HOECHST | |
| (5) NANOTUBES DE CARBONE SIMPLE PAROI de référence MRSW commercialisés par la société MER Corporation. | |

Le test est réalisé sur 20 modèles, 10 modèles avec application de la composition de l'invention et 10 modèles avec application de la composition témoin. Les caractéristiques cosmétiques des coiffures sont ensuite évaluées par un panel de 10 personnes.

Le panel indique à l'unanimité que l'application de la composition de l'invention engendre un effet net de moindre fléchissement des fibres sous l'effet de la pesanteur. Ce phénomène se traduit visuellement par un décollement des cheveux en racine et un apport en volume plus marqué. Après application de la composition de l'invention, les cheveux sont parfaitement individualisés et le toucher très proche des cheveux soumis à la composition témoin.

L'évaluation tactile et visuelle des diverses chevelures est répétée en suivant 1e même procédé et après avoir réalisé successivement deux shampooings (avec le shampooing DOP camomille), suivis d'un brushing.

Le panel indique à l'unanimité que les effets cosmétiques, et en particulier l'effet " volumateur " (effet de volume) conféré par l'emploi de la composition comprenant les nanotubes de carbone restent visibles après deux shampooings.

### Mise en évidence de l'effet " volumateur "

Afin de démontrer l'effet de moindre fléchissement des fibres kératiniques sous l'effet de la pesanteur après application de la composition de l'invention, la rigidité de la fibre en flexion est mesurée à l'aide de la méthode dite du " pendule de la souplesse ", avant et après traitement des fibres.

Le pendule utilisé est du type balançoire rigide qui bat la seconde. Ce pendule est constitué d'une barre de flexion en laiton poli. Cette barre est reliée à un axe par une tige de longueur L= 30 cm. Sur cette tige, à une distance L=18,5 cm de l'axe de rotation, est fixée une barre pesante de masse m=47g. l'énergie potentielle initiale du balancier est fixée par son angle d'inclinaison noté α.

Les éprouvettes de cheveux testées se présentent sous la forme d'un peigne de 39 fragments de cheveux de 11 mm de long collés parallèlement sur un support métallique.

La mesure s'effectue de la façon suivante : la barre en laiton est lâchée d'un angle α=30° sans vitesse initiale. A chaque passage au point bas (α=0°), la barre vient fléchir l'éprouvette de 39 cheveux et perd une partie de son énergie potentielle, et ce jusqu'à l'arrêt complet du pendule. Plus l'arrêt du pendule est rapide, plus les cheveux sont rigides. On considère que la perte d'énergie liée au frottement de la barre sur les cheveux est négligeable.

Pour mettre en évidence la rigidification de la fibre par l'application de la composition, une mesure est effectuée avant traitement puis une seconde mesure après traitement. On compare ensuite le nombre de battements nécessaires à l'arrêt du pendule avant et après traitement de l'éprouvette de cheveux. Le traitement des cheveux se réalise directement sur les cheveux montés sur les éprouvettes afin d'obtenir une plus grande sensibilité de la mesure. Les mesures ainsi que le séchage des éprouvettes de cheveux s'effectuent à température et humidité contrôlées (20°C et 45% HR).

**Résultats :** en appliquant la composition décrite dans l'exemple 1, et sur la base de 10 éprouvettes de cheveux mesurées, on observe que le traitement des cheveux par la composition de l'invention induit une diminution moyenne de 10% ± 2% du nombre de battements nécessaires à l'arrêt du pendule. Ces résultent montrent que l'application d'une composition comprenant des nanotubes de carbone parmet de rigidifier les fibres kératiniques, ce qui se traduit par le moindre fléchissement des fibres sous l'effet de la pesanteur, visualisé par un gonflement plus net de la chevelure.

## Revendications

1. Composition cosmétique, **caractérisée en ce qu'**elle comprend en association avec un milieu cosmétiquement acceptable des nanotubes constitués d'au moins un élément appartenant aux groupes II_{A}, III_{A}, IV_{A}, V_{A}, VIII, I_{B}, II_{B}, III_{B}, VI_{B} et VII_{B} de la classification périodique des éléments.

2. Composition selon la revendication 1, **caractérisée en ce que** les nanotubes sont constitués d'au mois un élément appartenant au groupe IV_{A} de la classification périodiques des éléments.

3. Composition selon la revendication 1 ou 2, **caractérisée en ce que** l'un des éléments constitutifs des nanotubes est le carbone.

4. Composition selon la revendication 3, **caractérisée en ce que** les nanotubes sont constitués totalement ou partiellement de molécules organiques.

5. Composition selon la revendication 3, **caractérisé en ce que** le squelette des nanotubes est constitué uniquement d'atomes de carbone.

6. Composition selon la revendication 5, **caractérisée en ce que** les nanotubes de carbone sont des nanotubes à simple paroi.

7. Composition selon la revendication 5, **caractérisée en ce que** les nanotubes de carbone sont des nanotubes multi-parois.

8. Composition selon l'une des revendications 5 à 7, **caractérisée en ce que** la surface des nanotubes de carbone est fonctionnalisée.

9. Composition selon la revendication 8, **caractérisée en ce que** la fonctionnalisation des nanotubes de carbone est réalisée par un mécanisme réactionnel du type substitution nucléophile, substitution électrophile, substitution radicalaire, addition, élimination, réarrangement, oxydation, réduction, réaction acido-basique, réaction électrochimique ou réaction photochimique.

10. Composition selon la revendication 8 ou 9, **caractérisée en ce que** la surface des nanotubes de carbone est fonctionnalisée par un ou plusieurs groupements choisis parmi les groupements carboxyliques, les fonctions hydrophobes, les amides à chaîne grasse, les oligomères, les polymères et les dendrimères.

11. Composition selon la revendication 8, **caractérisée en ce que** le ou les groupements fonctionnels sont susceptibles de créer avec les fibres kératiniques une ou plusieurs liaisons chimiques choisies parmi les interactions de type Van der Waals, les liaisons hydrogène, les liaisons ioniques et les liaisons covalentes.

12. Composition selon la revendication 11, **caractérisée en ce que** le ou les groupements fonctionnels susceptibles de créer avec les fibres kératiniques une ou plusieurs liaisons chimiques covalentes sont chosis parmi les groupements susceptibles de réagir avec les thiols, les disulfures, les acides carboxyliques, les alcools et les aminés.

13. Composition selon la revendications 12, **caractérisée en ce que** le ou les groupements fonctionnels susceptibles de réagir avec les thiols, les disulfures, les acides carboxyliques, les alcools et les aminés sont choisis parmi
- les époxydes,
- les groupements à cycle aziridine,
- les groupements vinyle et vinyle activé, tels que l'acrylonitrile, les esters acryliques et méthacrylique, l'acide et l'ester crotonique, les acides et esters cinnamique, le styrène et ses dérivés, le butadiène, les éthers de vinyle, les vinyle cétones, les esters maléiques, les maléimides, les vinyl sulfones, le vinyle cyanoacrylate,
- les acide carboxyliques et leurs dérivés comme les fonctions anhydride, chlorure d'acide, esters,
- les acétals, hémiacétals,
- les aminals, hémi-aminals,
- les cétones et alpha-hydroxycétones, alpha-halocétones,
- les lactones, thiolactones,
- l'isocyanate,
- le thiocyanate,
- les imines, '
- les imides (succinimides, glutimides), tels que le N-. hydroxysuccinimide ester,
- les imidates, .
- l'oxazine et l'oxazoline,
- l'oxazinium et l'oxazolinium,
- les halogénures d'alkyle ou d'aryle ou d'aralkyle, l'halogène étant choisi parmi l'iode, le brome ou le chlore,
- les halogénures de cycle insaturé, le cycle étant un cycle carboné ou un hétérocycle, tels que le chlorotriazine, le chloropyrimidine, le chloroquinoxaline, 1e chlorobenzotriazole,
- les halogénures de sulfonyle de formule RSO₂X, R étant un groupement alkyle, et X étant choisi parmi le fluor et 1e chlor,
- les dérivés du silicium tels que les alcoxysilanes, les silanols.

14. Composition selon l'une quelconque des revendications précédentes, **caractérisée en ce que** le diamètre des nanotubes est compris entre 1 et 300 nm et la longueur des nanotubes est comprise entre 10 nm et 10 mm.

15. Composition selon l'une quelconque des revendications précédentes, **caractérisée en ce que** les nanotubes sont présents dans la composition cosmétique dans des proportions en poids comprises entre 0,00001% et 30%, de préférence entre 0,0001% et 5%, et encore de préférence entre 0,001% et 1% du poids total de la composition.

16. Composition selon la revendication 8 ou 9, **caractérisée en ce qu'**elle contient au moins un tensioactif.

17. Composition selon la revendication 16, **caractérisée en ce que** le ou les tensioactifs sont choisis parmi les molécules amphiphiles, les oligomères amphiphiles, les dendrimères amphiphiles, les polymères amphiphiles et leurs mélanges.

18. Composition selon la revendication 16, **caractérisée en ce que** les tensio-actifs sont susceptibles de créer avec les fibres kératiniques une ou plusieurs liaisons chimiques covalentes par réaction avec les fonctions thiols, disulfures, acides carboxyliques, alcools et aminés des acides aminés composant la matière kératinique.

19. Composition selon la revendication 18, **caractérisée en ce que** les tensio-actifs comportent la fonction succinimidyl ester.

20. Composition selon l'une quelconque des revendications précédentes, **caractérisée en ce que** le milieu cosmétiquement acceptable de la solution comprenant les nanotubes est constitué par :
- de l'eau,
- des alcools aliphatiques ou aromatiques, de préférence l'éthanol, l'alcool benzylique, les alcools gras, les polyols modifiés ou non tels que le glycérol, le glycol, le propylène glycol, le dipropylène glycol, le butylène glycol, le butyle diglycol,
- des silicones, volatiles ou non,
- des huiles minérales, organiques ou végétales,
- des cires oxyéthylénées ou non, des paraffines, des alcanes et de préférence des alcanes de C₅ à C₁₀,
- des acides gras, des amides grasses, des esters gras et plus particulièrement des benzoates ou des salicylates d'alcool gras,
- de l'acétone, de la méthyléthylcétone, de l'acétate de méthyle, de l'acétate de butyle, de l'acétate d'étyle, du diméthoxyéthane, du diéthoxyéthane,
- leurs mélanges.

21. Composition selon l'une quelconque des revendications précédentes, **caractérisée en ce que** le milieu cosmétiquement acceptable se présente tel quel ou sous forme émulsionnée ou encapsulée.

22. Composition selon l'une quelconque des revendications précédentes, **caractérisée en ce que** la composition contient en outre un propulseur.

23. Composition selon la revendication 22, **caractérisé en ce que** le propulseur est constitué par des gaz comprimés ou liquéfiés choisis parmi l'air, le gaz carbonique, l'azote comprimé, un gaz soluble comme . le diméthyléther, les hydrocarbures halogénés (en particulier fluorés) ou non, et leurs mélanges.

24. Composition selon l'une quelconque des revendications précédentes, **caractérisée en ce que** la composition contient également des additifs cosmétiques usuels choisis parmi les agents réducteurs, les agents oxydants, les corps gras, les silicones, les agents épaississants, les adoucissants, les agents anti-mousse, les agents hydratants, les agents émollients, les agents alcalinisants, les plastifiants, les filtres solaires, les colorants directs ou d'oxydation, les pigments, les charges minérales, les argiles, les minéraux colloïdaux, les nacres, les parfums, les peptisants, les conservateurs, les polymères fixants ou non, les polymères conditionneurs, les protéines, les vitamines.

25. Composition selon l'une quelconque des revendications précédentes, **caractérisée en ce que** la composition se présente sous forme de lotion, spray, mousse, shampooing ou après-shampooing.

26. Utilisation cosmétique de nanotubes pour conférer du volume à des fibres kératiniques.

27. Utilisation selon la revendication 26, **caractérisé en ce que** les nanotubes sont constitués d'au moins un élément appartenant aux groupes II_{A}, III_{A}, IV_{A}, V_{A}, VIII, I_{B}, II_{B}, III_{B}, VI_{B} et VII_{B} de la classification périodique des éléments.

28. Utilisation selon la revendication 27, **caractérisée en ce que** les nanotubes sont constitués d'au mois un élément appartenant au groupe IV_{A} de la classification périodique des éléments.

29. Utilisation selon la revendication 27 ou 28, **caractérisée en ce que** l'un des éléments constitutifs des nanotubes est le carbone.

30. Utilisation selon la revendication 29, **caractérisée en ce que** les nanotubes sont constitués totalement ou partiellement de molécules organiques.

31. Utilisation selon la revendication 29, **caractérisé en ce que** le squelette des nanotubes est constitué uniquement d'atomes de carbone.

32. Utilisation selon la revendication 31, **caractérisée en ce que** les nanotubes de carbone sont des nanotubes à simple paroi.

33. Utilisation selon la revendication 31, **caractérisée en ce que** les nanotubes de carbone sont des nanotubes multi-parois.

34. Utilisation selon l'une des revendications 31 à 33, **caractérisée en ce que** la surface des nanotubes est fonctionnalisée.

35. Utilisation selon la revendication 34, **caractérisée en ce que** la fonctionnalisation des nanotubes de carbone est réalisée par un mécanisme réactionnel du type substitution nucléophile, substitution électrophile, substitution radicalaire, addition, élimination, réarrangement, oxydation, réduction, réaction acido-basique, réaction électrochimique ou réaction photochimique.

36. Utilisation selon la revendication 34 ou 35, **caractérisée en ce que** la surface des nanotubes de carbone est fonctionnalisée par un ou plusieurs groupements choisis parmi les groupements carboxyliques, les fonctions hydrophobes, les amides à chaîne grasse, les oligomères, les polymères et les dendrimères.

37. Utilisation selon l'une des revendications 34 à 36, **caractérisée en ce que** le ou les groupements fonctionnels sont susceptibles de créer avec les fibres kératiniques une ou plusieurs liaisons chimiques choisies parmi les interactions de type Van der Waals, les liaisons hydrogène, les liaisons ioniques et les liaisons covalentes.

38. Utilisation selon la revendication 37, **caractérisée en ce que** 1e ou les groupements fonctionnels susceptibles de créer avec les fibres kératiniques une ou plusieurs liaisons chimiques covalentes sont chosis parmi les groupements susceptibles de réagir avec les thiols, les disulfures, les acides carboxyliques, les alcools et les aminés.

39. Utilisation selon la revendication 38, **caractérisée en ce que** le ou les groupements fonctionnels susceptibles de réagir avec les thiols, les disulfures, les acides carboxyliques, les alcools et les aminés sont choisis parmi
- les époxydes,
- les groupements à cycle aziridine,
- les groupements vinyle et vinyle activé, tels que l'acrylonitrile, les esters acryliques et méthacrylique, l'acide et l'ester crotonique, les acides et esters cinnamique, le styrène et ses dérivés, 1e butadiène, les éthers de vinyle, les vinyle cétones, les esters maléiques, les maléimides, les vinyl sulfones, le vinyle cyanoacrylate,
- les acide carboxyliques et leurs dérivés comme les fonctions anhydride, chlorure d'acide, esters, ,
- les acétals, hémiacétals,
- les aminals, hémi-aminals,
- les cétones et alpha-hydroxycétones, alpha-halocétones,
- les lactones, thiolactones,
- l'isocyanate,
- le thiocyanate,
- les imines,
- les imides (succinimides, glutimides), tels que le N-hydroxysuccinimide ester,
- les imidates,
- l'oxazine ét l'oxazoline,
- l'oxazinium et l'oxazolinium,
- les halogénures d'alkyle ou d'aryle ou d'aralkyle, l'halogène étant choisi parmi l'iode, le brome ou le chlore,
- les halogénures de cycle insaturé, le cycle étant un cycle carboné ou un hétérocycle, tels que le chlorotriazine, le chloropyrimidine, le chloroquinoxaline, le chlorobenzotriazole,
- les halogénures de sulfonyle de formule RSO₂X, R étant un groupement alkyle, et X étant choisi parmi le fluor et le chlor,
- les dérivés du silicium tels que les alcoxysilanes, les silanols.

40. Utilisation selon l'une quelconque des revendications 26 à 39, **caractérisée en ce que** le diamètre des nanotubes est compris entre 1 et 300 nm et la longueur des nanotubes est comprise entre 10 nm et 10 mm.
